# EUROPEAN PATENT SPECIFICATION

(11) **EP 0 636 146 B1**
(45) Date of publication and mention of the grant of the patent: **30.07.1997**
(21) Application number: 93911459.1
(22) Date of filing: 13.04.1993
(51) Int. Cl.: C07K 7/56, A61K 38/12

(54) **TACHYKININ ANTAGONIST TRICYCLIC COMPOUNDS, PREPARATION OF SAME AND PHARMACEUTICAL COMPOSITIONS CONTAINING SUCH COMPOUNDS**
TRIZYKLISCHE TACHIKININ-ANTAGONISTEN, DEREN HERSTELLUNG UND DIESE ENTHALTENDE PHARMAZEUTISCHE PRÄPARATE
COMPOSES TRICYCLIQUES D'ANTAGONISTES DE TACHYKININE, PREPARATION DE CES DERNIERS ET COMPOSITIONS PHARMACEUTIQUES CONTENANT LESDITS COMPOSES

(30) Priority: 15.04.1992 IT FI920089
(43) Date of publication of application: 01.02.1995
(73) Proprietor: A. MENARINI INDUSTRIE FARMACEUTICHE RIUNITE S.R.L., I-50131 Firenze (IT); LABORATORI GUIDOTTI S.P.A., 56126 Pisa (IT); MALESCI ISTITUTO FARMACOBIOLOGICO S.p.A., I-50144 Firenze (IT)
(72) Inventor: PAVONE, Vincenzo, I-80129 Napoli (IT); LOMBARDI, Angelina, I-82034 Guardia Sanframondi (BN) (IT); PEDONE, Carlo, I-80121 Napoli (IT); MAGGI, Carlo Alberto, I-50141 Firenze (IT); QUARTARA, Laura, I-52037 San Sepolcro (AR) (IT)
(74) Representative: Gervasi, Gemma, Dr.
(86) International application number: EP9300893
(87) International publication number: WO9321227

(56) References cited:
- DE-A- 3 915 361
- GB-A- 2 216 529
- Chemical Abstracts, vol. 117, no. 11, 1992, Columbus, Ohio, US, S.L. HARBESON et al.
- Chemical Abstracts, vol. 116, no. 3, 1992, Columbus, Ohio, US, A.T. McKNIGHT et al.

## Description

### Field of the invention

The present invention refers to compounds having general formula (I) 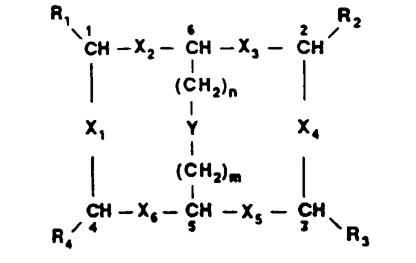 where:
X1, X2, X3, X4, X5, and X6, identical or different, are each selected out of the group consisting of -NR'-CO-, -CO-NR'-, where R' is H or C₁₋₃alkyl
Y is selected out of the group consisting of -CONR-, -NRCO-, -OCO-, -COO-, -CH₂-NR-, -NR-CH₂-, -SS-, -CH₂-CH₂-, cis or trans -CH=CH-, where R is H or C₁₋₃alkyl
R1, R2, R3, and R4 are a hydrophobic group
n and m, identical or different, are each a whole number from 1 to 4 the preparation of same and pharmaceutical compositions containing said compounds.

### State of the art

Tachyquinine antagonist compounds are known from literature. Among them, particularly interesting are the cyclic compounds [GB-A-2 216 529; McKnight, British Journal of Pharmacology, 104, 2 (1991); Gilon et al., Biopolymers, Vol. 31, 745-750 (1991); Harbeson et al., Peptides, Chemistry and Biology Proceedings 12th APS, 124 (1992), Ed. Escom].

Although the chemical formula of the compounds considered herein is considerably different from that of the compounds already known, the pharmacological activity of the former is equal to or even higher than that of the latter. Therefore, the claimed compounds may be regarded as valid alternatives.

### Detailed description of the invention

The present invention refers to new products of general formula (I) 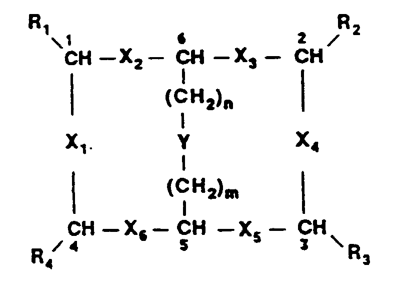 where:
X1, X2, X3, X4, X5, and X6, identical or different, are each selected out of the group consisting of -NR'-CO-, -CO-NR'-, where R' is H or C₁₋₃alkyl
Y is selected out of the group consisting of -CONR-, -NRCO-, -OCO-, -COO-, -CH₂-NR-, -NR-CH₂-, -SS-, -CH₂-CH₂-, cis or trans -CH=CH-, where R is H or C₁₋₃alkyl
R1, R2, R3, and R4 are each a hydrophobic group
n and m, identical or different, are each a whole number from 1 to 4 the processes for the preparation of same and pharmaceutical compositions containing such compounds.

As may be seen, the compounds as per formula (I) described above exhibit several chiral centres: it is understood that also the various enantiomers are an object of the present invention.

Hydrophobic groups R1, R2, R3, and R4 preferably consist of the side chains of hydrophobic amino acids, both natural and synthetic, or of the side chains of non-hydrophobic amino acids whose functional groups were derivatized in order to render them hydrophobic.

In particular, R1, R2, R3, and R4 may be selected out of the following groups:
a) linear or branched alkyl groups of the type CₙH₂ₙ₊₁ where n = 0, 1 to 4
b) linear or branched alkyl groups of the type CₙH₂ₙ-U-W where n = 1 to 4; U = O, CO, COO, CONH, S, guanidine, NH and W = H, hydrophobic group containing 1 to 10 carbon atoms
c) CH₂C₆H₃XY where X and Y, identical or different, are each H, halogen, OH, NH₂, CH₃ in an ortho or meta or para position of the benzene ring
d) CH₂C₆H₄X where X = OR, SR, NHR, where R = hydrophobic group containing 1 to 10 carbon atoms
e) C₆H₃XY where X and Y, identical or different, are each H, halogen, OH, NH₂, CH₃ in the ortho or meta or para position of the benzene ring
f) CH₂C₆H₁₁
g) 1-methyl-naphthyl, 2-methyl-naphthyl
h) CH₂-imidazole
i) CH₂-indole
l) CH₂-(furanyl-3-yl)
m) CH₂-(pyridyl-3-yl)
n) CH₂-(imidazolyl-3-yl)
o) an eventually substituted, -(CH₂)₃- group, which cyclizes with one of the two adjacent groups X to give the side chain of proline, hydroxyproline, dehydroproline.

In particular substituents R1, R2, R3, and R4 may be the side chains of hydrophobic natural amino acids selected out of the group consisting of: glycine, alanine, valine, leucine, isoleucine, methionine, phenylalanine, tyrosine, tryptophan, proline, histidine. R1, R2, R3, and R4 may also be the hydrophobic-derivatized side chains of non-hydrophobic amino acids selected out of the group consisting of: serine, threonine, cysteine, aspartic acid, asparagine, glutamic acid, glutamine, t-carboxyglutamic acid, arginine, ornithine, lysine.

R1, R2, R3, and R4 may also be the side chains of hydrophobic not natural amino acids selected out of the group consisting of: norleucine, norvaline, alloisoleucine, dehydroproline, hydroxyproline, cyclohexylglycine (Chg), α-amino-n-butyric acid (Aba), cyclohexylalanine (Cha), aminophenylbutyric acid (Pba), phenylalanines mono- and disubstituted in the ortho, meta, or para position of the aromatic ring with one or more of the following groups: C₁₋₁₀alkyl, C₁₋₁₀alcoxyl, halogen, β-2-thienylalanine, β-3-thienylalanine, β-2-furanylalanine, β-3-furanylalanine, β-2-pyridylalanine, β-3-pyridyiaianine, β-4-pyridyiaianine, β-(1-naphthyl)alanine, β-(2-naphthyl)alanine, O-alkylated derivatives of serine, threonine, tyrosine, S-alkylated cysteine, S-alkylated homocysteine, alkylated lysine, alkylated ornithine, 2,3-diaminopropionic acid.

Out of the products as per formula (I) as defined above, particularly preferred are the products in which:
1) R1 = -CH₂CH(CH₃)₂
   R2 = -CH₂C₆H₅ 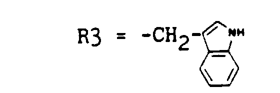
   R4 = -(CH₂)₂-SCH₃
   X1 = X2 = X3 = X4 = X5 = X6 = -CONH-
   Y = -CONH-
   wherein chiral carbon atoms exhibit L-configuration
2) Y = -NHCO-
   the other substituents being as defined under point (1)
3) R4= -CH₂-C₆H₁₁
   the other substituents being as defined under point (1)
4) Y = -NHCO-
   the other substituents being as defined under point (3)
5) R2 = R4 = -CH₂-C₆H₅ 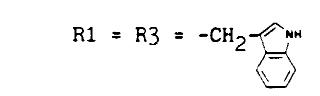 the other substituents being as defined under point (1)
6) Y = -NHCO-
   the other substituents being as defined under point (5)
7) Y = -SS-
   the other substituents being as defined under point (1)
8) Y = -CH₂-CH₂-
   the other substituents being as defined under point (1)
9) Y = -CH=CH- (cis)
   the other substituents being as defined under point (1)
10) Y = -CH=CH- (trans)
   the other substituents being as defined under point (1)
11) m = n = 1
   the other substituents being as defined under point (1)
12) m = 1, n = 2
   the other substituents being as defined under point (1)
13) m = 1, n = 3
   the other substituents being as defined under point (1)
14) m = 1, n = 4
   the other substituents being as defined under point (1)
15) m = 2, n = 1
   the other substituents being as defined under point (1)
16) m = 2, n = 2
   the other substituents being as defined under point (1)
17) m = 2, n = 3
   the other substituents being as defined under point (1)
18) m = 2, n = 4
   the other substituents being as defined under point (1)
19) X1 = X2 = X3 = X4 = X5 = X6 = -NHCO-
   the other substituents being as defined under point (1)
20) Y = -NHCO-
   the other substituents being as defined under point (19)
21) R4 = -CH₂-C₆H₁₁
   the other substituents being as defined under point (19)
22) Y = -NHCO-
   the other substituents being as defined under point (15)
23) R2 = R4 = -CH₂-C₆H₅ 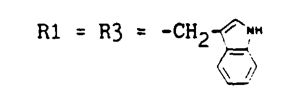 the other substituents being as defined under point (19)
24) Y = -NHCO-
   the other substituents being as defined under point (23)
25) Y = -SS-
   the other substituents being as defined under point (19)
26) Y = -CH₂-CH₂-
   the other substituents being as defined under point (19)
27) Y = -CH=CH- (cis)
   the other substituents being as defined under point (19)
28) Y = -CH=CH- (trans)
   the other substituents being as defined under point (19)
29) m = n = 1
   the other substituents being as defined under point (19)
30) m = 2; n = 4
   the other substituents being as defined under point (19)
31) the carbon atoms in positions 5 and 6 exhibit D-configuration
   all substituents being as defined under point (1)
32) all chiral carbon atoms exhibit D-configuration
   all substituents being as defined under point (1)

The compounds as per formula (I) covered by the invention can be prepared by known synthesis techniques, cf e.g. Schroeder et al., "The Peptides", Vol. 1, Academic Press, 1965; Bodansky et al., "Peptide Synthesis", Interscience Publishers, 1966; Barany and Merrifield, "The Peptides: Analysis, Synthesis, Biology", 2, Ch. 1, Academic Press, 1980.

The methods selected for the obtainment of the aforesaid products are the following:
i) synthesis in solution of the linear peptide chain by the coupling of suitably activated N-protected amino acids with an amino acid or a C-protected peptide chain, with intermediates isolation, followed by selective deprotection of C- and N-terminal chains, cyclization in organic polar solvents dilute solution, selective deprotection of the side chains and their cyclization in organic polar solvents dilute solution (cf also Bodansky-Bodansky, "The procedure of peptide synthesis", Springer Verlag, 1984).
ii) peptide chain solid phase synthesis from C-terminal end to N-terminal end on an insoluble polymer support, cyclization in the solid phase of previously deprotected side chains, followed by detachment from the polymer support by hydrolysis in anhydrous hydrofluoric acid containing suitable scavengers or in trifluoracetic acid containing suitable scavengers and cyclization of monocyclic peptide in organic polar solvents dilute solution. The process described above can alternatively consist of peptide chain solid phase synthesis from C-terminal end to N-terminal end on an insoluble polymer support, detachment from the polymer support by hydrolysis in anhydrous hydrofluoric acid containing suitable scavengers or in trifluoracetic acid containing suitable scavengers, cyclization of C-terminal and N-terminal ends in organic polar solvents dilute solution, deprotection of side chains and their cyclization in organic polar solvents dilute solution (cf the method described by Atherton et al. in Bioorganic Chemistry, 8, 351, 1979; by Merrifield in J.Am.Chem.Soc., 85, 2149-2154 (1963)). The first cyclization reaction can be carried out directly on the insoluble solid support (cf A.M. Felix et al., Int. J. Pep.Prot.Res., 31, 231, 1988; P. Rovero et al., Tetrahedron Letters, 32, 23, 2639 (1991), whereas the second cyclization can be carried out also in solution according to the procedures well known in the chemistry of peptide linkages (cf Kopple K.D., J. Pharmaceutical Sci., 61, 1345, 1972). According to a particular method, the desired product may be obtained with PAM-resin (phenylacetoamidomethyl resin - A.R. Mitchell et al., J. Org. Chem., 43, 2845, 1978) functionalized with a Boc group protected amino acid at the N-terminal end. The amino acids directly bound to the resin are preferably the hydrophobic ones, such as Leu. After introduction of the other amino acids in the sequence, a first cyclization may be carried out reaching the side chains of the preferred aminoacids after their selective deprotection and activation. The monocyclic peptide can be removed by liquid hydrofluoric acid. The free peptide at N- and C-terminal ends can be further cyclized according to traditional synthesis methods.

The compounds as per formula (I) defined above proved to be more effective tachyquinine antagonists than other analogous antagonists; it follows that - compared with the known products - they may be administered at lower dose levels.

Therefore, they are suitable for the treatment of arthritis, asthma, inflammations, tumoral growth, gastrointestinal hypermotility, Huntington's disease, neuritis, neuralgia, migraine, hypertension, incontinence of urine, urticaria, carcinoid syndrome symptoms, influenza and cold.

The compounds as per formula (I) covered by the invention are suitable for therapeutic administration to animals and man by the parenteral. oral, inhalatory, and sublingual ways, with pharmacological effects matching the described properties. In case of parenteral administration (intravenous, intramuscular, intradermal), the compounds to be used are sterile solutions or freeze-dried preparations. In case of oral administration, preparations such as tablets, capsules and syrups are conveniently used. Suitable dosed ointments and creams are utilizable by the dermic way. In case of nasal instillation, inhalation, and sublingual administration, the compounds to be used are respectively aqueous solutions, aerosol preparations, or capsules.

Active ingredient doses in the aforesaid compositions range from 0.1 to 10 mg/kg body weight.

### EXAMPLE 1

### Preparation of cyclo(Met-Asp-Trp-Phe-Leu)

[Compound as per formula (I) where: Y = X1 = X2 = X3 = X4 = X5 = X6 = -NHCO-; m = n = 1; R1 = -CH₂-CH(CH₃)₂; 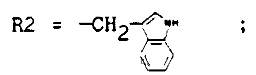 R3 = -CH₂-C₆H₅; R4 = -CH₂-CH₂-SCH₃; and carbon atoms C₁, C₂, C₃, C₄, C₅, C₆ have L-configuration]

### Compound (1)

### a) Synthesis of the monocyclic peptide having the following sequence: H-Met-Asp-Trp-Phe-Dpr-Leu-OH

0.625 Grams Boc-Leu-OCH₂-PAM resin (Applied Biosystem, USA, 0.8 meq/g), equal to 0.5 mmoles of amine groups, is fed an Applied BioSystem 430A (Foster City, CA, USA) semi-automatic peptide synthesis reactor. The Boc group is hydrolyzed with 33% TFA in DCM for 1.5 min. and with 50% TFA in DCM for 18.5 min.; then it is neutralized with in DMF with 10% DIEA solution for 2 min. The following residues are made to react in the same order, in the quantities indicated in brackets: Boc-Dpr(Fmoc)-OH (0.852 g), Boc-Phe-OH (0.512 g), Boc-Trp(CHO)-OH (0.664 g), Boc-Asp(OFm)-OH (0.822 g).

The first acylation lasts 1 hour. The resin is washed and the reaction is ninhydrin-tested by the Kaiser method. In case of a negative response, the Boc group is hydrolyzed as described above, before the subsequent amino acid coupling. Acylation with Boc-Dpr(Fmoc)-OH is performed by adding an amino acid (2 mmoles) and PyBop (2 mmoles) solution in DMF to the deprotected resin. Boc-Phe-OH and Boc-Trp(CHO)-OH are coupled in the form of symmetric anhydride by dissolving 2 mmoles amino acid in 5 ml dichloromethane. The solution temperature is brought to 0°C and 1 ml of a 0.5 M solution of dicyclohexyl carbodiimide in dichloromethane is added.

After 15 minutes, dicyclohexylurea is filtered and the resulting solution is added to the deprotected resin. Boc-Asp(OFm)-OH coupling is performed by adding the deprotected resin with an amino acid (2 mmoles) and HOBt (2 mmoles) solution in DMF; after 2 minutes, the suspension is added with a 0.5 M solution of DCC in DCM (4 ml). The fluorenyl groups on Asp and Dpr side chains are removed by treatment with a 20% (v/v) piperidine solution in DMF (15 ml twice for 3 and 7 min.). The condensation between β-amino and β-carboxyl groups is carried out with a 0.25 M solution of PyBop in DMF (3 equivalents) in the presence of DIEA (6 equivalents) until negative response of the Kaiser Test.

Activated Boc-Met-OH (0.498 g) in the form of symmetric anhydride is coupled and, after terminal amine group deprotection, the formyl group of tryptophan is deprotected by treatment with 120 ml of 1 M solution of TMSiBr and 1 M solution of thioanisole in TFA in the presence of 1.2 ml m-cresol and 1.2 ml EDT. After 1 hour at 0°C, the solution is filtered, the resin washed with TFA and dried. The dry resin is placed in a Teflon reactor with 1 ml anisole and 1 ml dimethyl sulphide. The mixture temperature is brought to -50°C and 10 ml hydrofluoric acid is distilled therein; then the mixture is kept under stirring for 60 min. in an ice bath. Hydrofluoric acid is removed by nitrogen blowing. The raw product is dried under suction for about 2 hours, is washed with ethyl ether (15 ml twice), extracted in 50% acetic acid (15 ml three times), and filtered in a porous filter funnel to remove the exhaust resin. The resulting solution is diluted with water and freeze-dried. Finally, the peptide is purified by reversed phase chromatography and characterized by analytical HPLC on Varian LC Star 9010 Vydac C18 0.46 x 25 cm column with a linear acetonitrile gradient containing 0.1% (v/v) trifluoracetic acid (phase B) vs. 0.1% (v/v) aqueous trifluoracetic acid (phase A), as 5% to 70% phase B, in 50 min., at a rate of 1 ml/min., with 210 nm UV monitoring. Retention time (Rt) = 26.3'; chromatographic purity > 99%.
FAB-MS: (M + H)⁺ = 779.

### b) Cyclization of (a)

70 mg product (a) obtained as above is dissolved in 90 ml DMF. The solution is added with 47 mg PyBOP plus 20 µl DIEA. The resulting solution is kept under stirring at 0°C for 18 hours, then DMF is removed under vacuum and the resulting mixture freeze-dried. Compound (1) is purified by reversed phase liquid chromatography and characterized by analytical HPLC, on Varian LC Star 9010 Vydac C18 0.46 x 25 cm column with a linear acetonitrile gradient containing 0.1% (v/v) trifluoracetic acid (phase B) vs. 0.1% (v/v) aqueous trifluoracetic acid (phase A), as 5% to 70% phase B, in 50 min., at a rate of 1 ml/min., with 210 nm UV monitoring. Retention time (Rt) = 29.5'; chromatographic purity > 99%.
FAB-MS: (M + H)⁺ = 761.

### BIOLOGICAL ACTIVITY

The capacity of the products described in the present invention to interact with the neuroquinine A receptor as agonists or antagonists was assessed using a preparation characterized by the fact that the biological response produced by tachyquinines and correlated peptides was exclusively determined by the neuroquinine A receptor (receptor NK-2). The said preparation consisted of isolated rabbit pulmonary artery affected by a dose-dependent contraction brought about by tachyquinines (Rovero et al., Neuropeptides, 13, 263-270, 1989). The determination of the peptide activity in the test preparation was based on the use of an NKA concentration (3 nM) causing a response equal to 45% of max. response. The peptides considered herein were added to the preparation in growing concentrations. Their activity was assessed as inhibition of response to NKA.

By way of example, compound 1 tested at a concentration of 1 M caused 100% inhibition of response to neuroquinine A in isolated rabbit pulmonary artery.

The capacity of the products described herein to interact with the P substance receptor (receptor NK-1) was assessed through an in vitro test, where the biological response produced by tachyquinines and correlated peptides was exclusively determined by the P substance receptor. The test preparation consisted of isolated guinea pig ileum affected by a dose-dependent contraction brought about by tachyquinines (Lee et al., Schmied. Arch. Pharmacol., 318, 281-287, 1982). The determination of the activity of the products as per the present invention in the test preparation was based on the use of an SP methyl ester concentration (10 nm) causing a response equal to 45% of max. response (S. Dion et al., Life Sci., 41, 2269-2278, 1987). The products considered herein were added to the preparation in growing concentrations. Their activity was assessed as inhibition of response to SP with satisfactory results.

By way of example, product 1 tested at a concentration of 10 mM caused 100% inhibition of response to SP methyl ester.

### Abbreviations used

For the nomenclature and abbreviations of amino acids, reference is made to the rules issued by the IUPAC-IUB Joint Commission on Biochemical Nomenclature (Eur. J. Biochem., 1984, 138:9); unless otherwise specified, amino acids are considered in the L-configuration.

The other abbreviations used are the following:

Boc = tert-butyloxycarbonyl; DCM = dichloromethane; BOP = benzotriazolyl-N-oxytri(dimethylaminophosphonium) hexafluorophosphate, Dpr = 2,3-diaminopropionic acid; DCC = N-N'-dicyclohexyl carbodiimide; DCU = N-N' dicyclohexylurea; DIEA = diisopropylethylamine, DMF = N-N' dimethylformamide; EDT = ethandithiol; FAB-MS = fast atoms bombardment mass spectrometry; Fmoc = 9-fluorenylmethyloxycarbonyl, HOBt = 1-hydroxybenzotriazole; HPLC = high pressure liquid chromatography; iPrOH = isopropanol; PAM = phenylacetamidomethyl; NKA = neuroquinine A; SP = P substance; PIP = piperidine; TFA = trifluoracetic acid; For = formyl; Me = methyl; Ac = acetyl; Fm = fluorenylmethyl; PyBop = benzotriazole-1-yl-oxypyrrolidinephosphonium hexafluorophosphate.

## Claims (Claims for the following Contracting State(s): AT, BE, CH, DE, DK, FR, GB, GR, IE, IT, LI, LU, MC, NL, PT, SE)

1. Products of general formula (I) 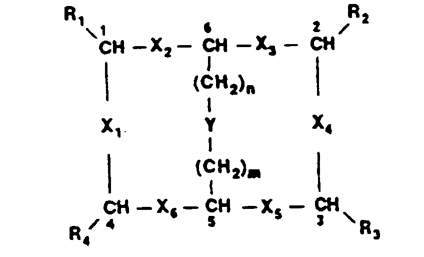 where:
X1, X2, X3, X4, X5, and X6, identical or different, are each selected out of the group consisting of -NR'-CO-, -CO-NR'-, where R' is chosen in the group consisting of H, C₁₋₃alkyl
Y is selected out of the group consisting of -CONR-, -NRCO-, -OCO-, -COO-, -CH₂-NR-, -NR-CH₂-, -SS-, -CH₂-CH₂-, cis or trans -CH=CH-, where R is chosen in the group consisting of H, C₁₋₃alkyl R1, R2, R3. and R4 are each a hydrophobic group
n and m, identical or different, are each a whole number from 1 to 4.

2. The compounds of formula (I) according to claim 1 wherein R1, R2, R3, and R4 are selected out of the following groups:
a) linear or branched alkyl groups of the type CₙH₂ₙ₊₁ where n = 0, 1 to 4
b) linear or branched alkyl groups of the type CₙH₂ₙ-U-W where n = 1 to 4; U = O, CO, COO, CONH, S, guanidine, NH and W = H, hydrophobic group containing 1 to 10 carbon atoms
c) CH₂C₆H₃XY where X and Y, identical or different, are each H, halogen, OH, NH₂, CH₃ in the ortho or meta or para position of the benzene ring
d) CH₂C₆H₄X where X = OR, SR, NHR, where R = hydrophobic group containing 1 to 10 carbon atoms
e) C₆H₃XY where X and Y, identical or different, are each H, halogen, OH, NH₂, CH₃ in the ortho or meta or para position of the benzene ring
f) CH₂C₆H₁₁
g) 1-methyl-naphthyl, 2-methyl-naphthyl
h) CH₂-imidazole
i) CH₂-indole
l) CH₂-(furanyl-3-yl)
m) CH₂-(pyridyl-3-yl)
n) CH₂-(imidazolyl-3-yl)
o) an eventually substituted, -(CH₂)₃- group, which cyclizes with one of the two adjacent groups X to give the side chain of proline, hydroxyproline, dehydroproline the other substituents being as defined in claim 1.

3. The compounds of formula (I) according to claim 2 wherein: R1, R2, R3, and R4 are the side chains of amino acids selected out of the group consisting of: glycine, alanine, valine, leucine, isoleucine, methionine, phenylalanine, tyrosine, tryptophan, proline, histidine, norleucine, norvaline, alloisoleucine, dehydroproline, hydroxyproline, cyclohexylglycine (Chg), α-amino-n-butyric acid (Aba), cyclohexylalanine (Cha), aminophenylbutyric acid (Pba), phenylalanine mono- and disubstituted in the ortho, meta, or para position of the aromatic ring with one or more of the following groups: C₁₋₁₀alkyl, C₁₋₁₀alcoxyl, halogen, β-2-thienylalanine, β-3-thienylalanine, β-2-furanylalanine, β-3-furanylalanine, β-2-pyridylalanine, β-3-PYridylalanine, β-4-pyridylalanine, β-(1-naphthyl)alanine, β-(2-naphthyl)alanine, O-alkylated derivatives of serine, threonine, tyrosine, S-alkylated cysteine, S-alkylated homocysteine, alkylated lysine, alkylated ornithine, 2,3-diaminopropionic acid;
or they are the side chains of non-hydrophobic amino acids whose functional groups where derivatized in order to render them hydrophobic, selected out of the group consisting of: serine, threonine, cysteine, aspartic acid, asparagine, glutamic acid, glutamine, t-carboxyglutamic acid, arginine, ornithine, lysine.

4. The compounds of formula (I) according to claim 3 wherein:
R1 = -CH₂CH(CH₃)₂
R2 = -CH₂C₆H₅ 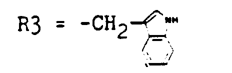
R4 = -(CH₂)₂-SCH₃
X1 = X2 = X3 = X4 = X5 = X6 = -CONH-
Y = -CONH-
wherein chiral carbon atoms exhibit L-configuration.

5. The compounds of formula (I) according to claim 3 wherein:
Y = -NHCO-
the other substituents being as defined in claim 4.

6. The compounds of formula (I) according to claim 3 wherein:
R4 = -CH₂-C₆H₁₁
the other substituents being as defined in claim 4.

7. The compounds of formula (I) according to claim 3 wherein:
Y = -NHCO-
the other substituents being as defined in claim 6.

8. The compounds of formula (I) according to claim 3 wherein:
R2 = R4 = -CH₂-C₆H₅ 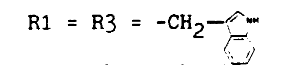
the other substituents being as defined in claim 4.

9. The compounds of formula (I) according to claim 3 wherein:
Y = -NHCO-
the other substituents being as defined in claim 8.

10. The compounds of formula (I) according to claim 3 wherein:
Y = -SS-
the other substituents being as defined in claim 4.

11. The compounds of formula (I) according to claim 3 wherein:
Y = -CH₂-CH₂-
the other substituents being as defined in claim 4.

12. The compounds of formula (I) according to claim 3 wherein:
Y = -CH=CH- (cis)
the other substituents being as defined in claim 4.

13. The compounds of formula (I) according to claim 3 wherein:
Y = -CH=CH- (trans)
the other substituents being as defined in claim 4.

14. The compounds of formula (I) according to claim 3 wherein:
Y = -CH₂NH-
the other substituents being as defined in claim 4.

15. The compounds of formula (I) according to claim 3 wherein:
Y = -NHCH₂-
the other substituents being as defined in claim 4.

16. The compounds of formula (I) according to claim 3 wherein:
m = n = 1
the other substituents being as defined in claim 4.

17. The compounds of formula (I) according to claim 3 wherein:
m = 2, n = 4
the other substituents being as defined in claim 4.

18. The compounds of formula (I) according to claim 3 wherein:
X1 = X2 = X3 = X4 = X5 = X6 = -NHCO-
the other substituents being as defined in claim 4.

19. The compounds of formula (I) according to claim 3 wherein:
Y = -NHCO-
the other substituents being as defined in claim 18.

20. The compounds of formula (I) according to claim 3 wherein:
R4 = -CH₂-C₆H₁₁
the other substituents being as defined in claim 18.

21. The compounds of formula (I) according to claim 3 wherein:
Y = -NHCO-
the other substituents being as defined in claim 20.

22. The compounds of formula (I) according to claim 3 wherein:
R2 = R4 = -CH₂-C₆H₅ 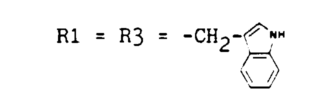
the other substituents being as defined in claim 18.

23. The compounds of formula (I) according to claim 3 wherein:
Y = -NHCO-
the other substituents being as defined in claim 22.

24. The compounds of formula (I) according to claim 3 wherein:
Y = -SS-
the other substituents being as defined in claim 18.

25. The compounds of formula (I) according to claim 3 wherein:
Y = -CH₂-CH₂-
the other substituents being as defined in claim 18.

26. The compounds of per formula (I) according to claim 3 wherein:
Y = -CH=CH- (cis)
the other substituents being as defined in claim 18.

27. The compounds of formula (I) according to claim 3 wherein:
Y = -CH=CH- (trans)
the other substituents being as defined in claim 18.

28. The compounds of formula (I) according to claim 3 wherein:
m = n = 1
the other substituents being as defined in claim 18.

29. The compounds of formula (I) according to claim 3 wherein:
m = 1; n = 2
the other substituents being as defined in claim 18.

30. The compounds of formula (I) according to claim 3 wherein:
m = 1; n = 3
the other substituents being as defined in claim 18.

31. The compounds of formula (I) according to claim 3 wherein:
m = 1; n = 4
the other substituents being as defined in claim 18.

32. The compounds of formula (I) according to claim 3 wherein:
m = 2; n = 1
the other substituents being as defined in claim 18.

33. The compounds of formula (I) according to claim 3 wherein:
m = 2; n = 3
the other substituents being as defined in claim 18.

34. The compounds of formula (I) according to claim 3 wherein:
m = 2; n = 2
the other substituents being as defined in claim 18.

35. The compounds of formula (I) according to claim 3 wherein:
m = 2; n = 4
the other substituents being as defined in claim 18.

36. The compounds of formula (I) according to claim 3 wherein the carbon atoms in positions 5 and 6 exhibit D-configuration, all substituents being as defined in claim 4.

37. The compounds of formula (I) according to claim 3 wherein all chiral carbon atoms exhibit D-configuration, all substituents being as defined in claim 4.

38. The pharmaceutical compositions containing compounds of formula (I) according to claim 1 mixed with suitable carriers.

39. The pharmaceutical compositions according to claim 38 for use as tachyquinine antagonists.

40. The compositions according to claim 38 for the treatment of arthritis, asthma, inflammations, tumoral growth, gastrointestinal hypermotility , Huntington's disease, neuritis , neuralgia, migraine, hypertension. incontinence of urine, urticaria. carcinoid syndrome symtoms, influenza and cold.

## Claims (Claims for the following Contracting State(s): ES)

1. Process for the prparation of compounds of general formula (I) 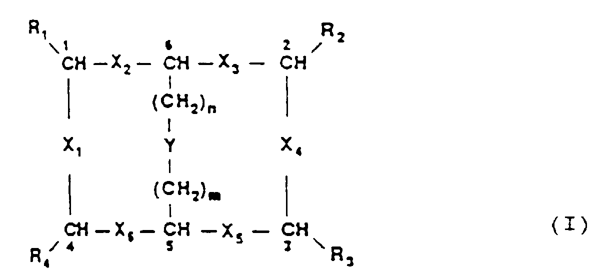 where:
X1, X2, X3, X4, X5, and X6, identical or different, are each selected out of the group consisting of -NR'-CO-, -CO-NR'-, where R' is chosen in the group consisting of H, C₁₋₃alkyl Y is selected out of the group consisting of -CONR-, -NRCO-, -OCO-, -COO-, -CH₂-NR-, -NR-CH₂-, -SS-, -CH₂-CH₂-, cis or trans -CH=CH-, where R is chosen in the group consisting of H, C₁₋₃alkyl.
R1, R2, R3, and R4 are each a hydrophobic group
n and m, identical or different, are each a whole number from 1 to 4.
wherein:
a peptide chain is synthetised in solid phase from C-terminal end to N-terminal end on an insoluble polymer support, the peptide chain is deprotected and cyclized in solid phase therefater detached from the polymer support by hydrolysis in anhydrous hydrofluoric acid or trifluoroacet acid, containing suitable scavengers, and finally the monocyclic peptide is cyclized in organic polar solvents dilute solution.

2. Process according to claim 1 wherein R1, R2, R3, and R4 are selected out of the following groups:
a) linear or branched alkyl groups of the type CₙH₂ₙ₊₁ where
n = 0, 1 to 4
b) linear or branched alkyl groups of the type CₙH₂ₙ-U-W
where n = 1 to 4; U = O, CO, COO, CONH, S, guanidine, NH and W = H, hydrophobic group containing 1 to 10 carbon atoms
c) CH₂C₆H₃XY where X and Y, identical or different, are each
H, halogen, OH, NH₂, CH₃ in the ortho or meta or para position of the benzene ring
d) CH₂C₆H₄x where X = OR, SR, NHR, where R = hydrophobic group containing 1 to 10 carbon atoms
e) C₆H₃XY where X and Y, identical or different, are each H, halogen, OH, NH₂, CH₃ in the ortho or meta or para position of the benzene ring
f) CH₂C₆H₁₁
g) 1-methyl-naphthyl, 2-methyl-naphthyl
h) CH₂-imidazole
i) CH₂-indole
l) CH₂-(furanyl-3-yl)
m) CH₂-(pyridyl-3-yl)
n) CH₂-(imidazolyl-3-yl)
o) an eventually substituted, -(CH₂)₃- group, which cyclizes with one of the two adjacent groups X to give the side chain of proline, hydroxyproline, dehydroproline the other substituents being as defined in claim 1.

3. Process according to claim 2 wherein: R1, R2, R3, and R4 are the side chains of amino acids selected out of the group consisting of: glycine, alanine, valine, leucine, isoleucine, methionine, phenylalanine, tyrosine, tryptophan, proline, histidine, norleucine, norvaline, alloisoleucine, dehydroproline, hydroxyproline, cyclohexylglycine (Chg), α-amino-n-butyric acid (Aba), cyclohexylalanine (Cha), aminophenylbutyric acid (Pba), phenylalanine mono- and disubstituted in the ortho, meta, or para position of the aromatic ring with one or more of the following groups: C₁₋₁₀alkyl, C₁₋₁₀alcoxyl, halogen, β-2-thienylalanine, β-3-thienylalanine, β-2-furanylalanine, β-3-furanylalanine, β-2-pyridylalanine, β-3-pyridylalanine, β-4-pyridylalanine, β-(1-naphthyl)alanine, β-(2-naphthyl)alanine, O-alkylated derivatives of serine, threonine, tyrosine, S-alkylated cysteine, S-alkylated homocysteine, alkylated lysine, alkylated ornithine, 2,3-diaminopropionic acid; or they are the side chains of non-hydrophobic amino acids whose functional groups where derivatized in order to render them hydrophobic, selected out of the group consisting of: serine, threonine, cysteine, aspartic acid, asparagine, glutamic acid, glutamine, t-carboxyglutamic acid, arginine, ornithine, lysine.

4. Process according to claim 3 wherein in the compounds of formula (I):
R1 = -CH₂CH(CH₃)₂
R2 = -CH₂C₆H₅ 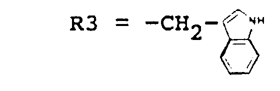
R4 = -(CH₂)₂-SCH₃
X1 = X2 = X3 = X4 = X5 = X6 = -CONH-
Y = -CONH-
wherein chiral carbon atoms exhibit L-configuration.

5. Process according to claim 3 wherein in the compounds of formula (I):
Y = -NHCO-
the other substituents being as defined in claim 4.

6. Process according to claim 3 wherein in the compounds of formula (I):
R4 = -CH₂-C₆H₁₁
the other substituents being as defined in claim 4.

7. Process according to claim 3 wherein in the compounds of formula (I):
Y = -NHCO-
the other substituents being as defined in claim 6.

8. Process according to claim 3 wherein in the compounds of formula: (I)
R2 = R4 = -CH₂-C₆H₅ 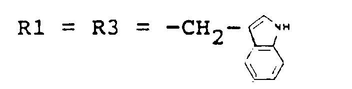
the other substituents being as defined in claim 4.

9. Process according to claim 3 wherein in the compounds of formula : (I)
Y = -NHCO-
the other substituents being as defined in claim 8.

10. Process according to claim 3 wherein in the compounds of formula :(I)
Y = -SS-
the other substituents being as defined in claim 4.

11. Process according to claim 3 wherein in the compounds of formula (I):
Y = -CH₂-CH₂-
the other substituents being as defined in claim 4.

12. Process according to claim 3 wherein in the compounds of formula : (I)
Y = -CH=CH- (cis)
the other substituents being as defined in claim 4.

13. Process according to claim 3 wherein in the compounds of formula (I):
Y = -CH=CH- (trans)
the other substituents being as defined in claim 4.

14. Process according to claim 3 wherein in the compounds of formula (I):
Y = -CH₂NH-
the other substituents being as defined in claim 4.

15. Process according to claim 3 wherein in the compounds of formula (I):
Y = -NHCH₂-
the other substituents being as defined in claim 4.

16. Process according to claim 3 wherein in the compounds of formula (I):
m = n = 1
the other substituents being as defined in claim 4.

17. Process according to claim 3 wherein in the compounds of formula (I):
m = 2, n = 4
the other substituents being as defined in claim 4.

18. Process according to claim 3 wherein in the compounds of formula (I):
X1 = X2 = X3 = X4 = X5 = X6 = -NHCO-
the other substituents being as defined in claim 4.

19. Process according to claim 3 wherein in the compounds of formula (I):
Y = -NHCO-
the other substituents being as defined in claim 18.

20. Process according to claim 3 wherein in the compounds of formula (I):
R4 = -CH₂-C₆H₁₁
the other substituents being as defined in claim 18.

21. Process according to claim 3 wherein in the compounds of formula (I):
Y = -NHCO-
the other substituents being as defined in claim 20.

22. Process according to claim 3 wherein in the compounds of formula (I):
R2 = R4 = -CH₂-C₆H₅ 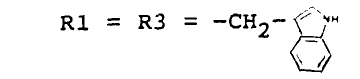
the other substituents being as defined in claim 18.

23. Process according to claim 3 wherein in the compounds of formula (I):
Y = -NHCO-
the other substituents being as defined in claim 22.

24. Process according to claim 3 wherein in the compounds of formula (I):
Y = -SS-
the other substituents being as defined in claim 18.

25. Process according to claim 3 wherein in the compounds of formula (I):
Y = -CH₂-CH₂-
the other substituents being as defined in claim 18.

26. Process according to claim 3 wherein in the compounds of formula (I):
Y = -CH=CH- (cis)
the other substituents being as defined in claim 18.

27. Process according to claim 3 wherein in the compounds of formula (I):
Y = -CH=CH- (trans)
the other substituents being as defined in claim 18.

28. Process according to claim 3 wherein in the compounds of formula (I):
m = n = 1
the other substituents being as defined in claim 18.

29. Process according to claim 3 wherein in the compounds of formula (I):
m = 1; n = 2
the other substituents being as defined in claim 18.

30. Process according to claim 3 wherein in the compounds of formula (I):
m = 1; n = 3
the other substituents being as defined in claim 18.

31. Process according to claim 3 wherein in the compounds of formula (I):
m = 1; n = 4
the other substituents being as defined in claim 18.

32. Process according to claim 3 wherein in the compounds of formula (I):
m = 2; n = 1
the other substituents being as defined in claim 18.

33. Process according to claim 3 wherein in the compounds of formula (I):
m = 2; n = 3
the other substituents being as defined in claim 18.

34. Process according to claim 3 wherein in the compounds of formula (I):
m = 2; n = 2
the other substituents being as defined in claim 18.

35. Process according to claim 3 wherein in the compounds of formula (I):
m = 2; n = 4
the other substituents being as defined in claim 18.

36. Process according to claim 3 wherein in the compounds of formula (I)
wherein the carbon atoms in positions 5 and 6 exhibit D-configuration,
all substituents being as defined in claim 4.

37. Process according to claim 3 wherein in the compounds of formula (I)
wherein all chiral carbon atoms exhibit D-configuration, all substituents being as defined in claim 4.

38. The pharmaceutical compositions containing compounds of formula (I) according to claim 1 mixed with suitable carriers such as herein described.

39. The pharmaceutical compositions according to claim 38 for use as tachyquinine antagonists.

40. The compositions according to claim 38 for the treatment of arthritis, asthma, inflammations, tumoral growth, gastrointestinal hypermotility, Huntington's disease, neuritis, neuralgia, migraine, hypertension, incontinence of urine, urticaria, carcinoid syndrome symptoms, influenza and cold.

## Patentansprüche (Patentansprüche für folgende(n) Vertragsstaat(en): AT, BE, CH, DE, DK, FR, GB, GR, IE, IT, LI, LU, MC, NL, PT, SE)

1. Produkte der allgemeinen Formel (I) 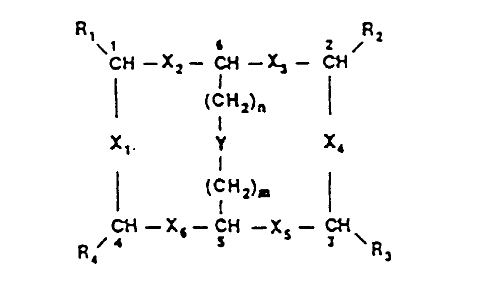 wobei
X₁, X₂, X₃, X₄, X₅ und X₆, die gleich oder verschieden sein können, je aus der Gruppe ausgewählt werden, bestehend aus -NR'-CO-, -CO-NR'-, wobei R' aus der Gruppe, bestehend aus H, C₁₋₃-Alkyl, ausgewählt wird;
Y ausgewählt wird aus der Gruppe, bestehend aus -CONR-, -NRCO-, -OCO-, -COO-, -CH₂-NR-, -NR-CH₂, -SS-, -CH₂-CH₂-, cis- oder trans- -CH=CH-, wobei R ausgewählt aus der Gruppe, bestehend aus H, C₁₋₃-Alkyl;
R₁, R₂, R₃ und R₄ je eine hydrophobe Gruppe sind;
n und m, die gleich oder verschieden sein können, je ein Ganzzahliges von 1 bis 4 sind.

2. Verbindungen der Formel (I) nach Anspruch 1, wobei R₁, R₂, R₃ und R₄ aus den nachfolgenden Gruppen ausgewählt werden:
a) linearen oder verzweigten Alkylgruppen des Typs CₙH₂ₙ₊₁, wobei n=0, 1 bis 4 ist;
b) linearen oder verzweigten Alkylgruppen des Typs CₙH₂ₙ-U-W, wobei n = 1 bis 4; U = O, CO, COO, CONH, S, Guanidin, NH und W=H, einer hydrophoben Gruppe mit 1 - 10 Kohlenstoffatomen;
c) CH₂C₆H₃XY, wobei X und Y, die gleich oder unterschiedlich sein können, je H, ein Halogen, OH, NH₂, CH₃ sind und in Ortho- oder Meta- oder Para-Stellung zum Benzolring stehen können;
d) CH₂C₆H₄X, wobei X = OR, SR, NHR, wobei R= eine hydrophobe Gruppe mit 1 - 10 Kohlenstoffatomen ist;
e) C₆H₃XY, wobei X und Y, die gleich oder verschieden sein können, je H, ein Halogen, OH, NH₂, CH₃ sind und in Ortho- oder Meta- oder Para-Position zum Benzolring stehen können;
f) CH₂C₆H₁₁
g) 1-Methyl-naphthyl, 2-Methyl-naphthyl,
h) CH₂-Imidazol,
i) CH₂-Indol,
l) CH₂-(Furanyl-3-yl),
m) CH₂-(Pyridyl-3-yl),
n) CH₂-(Imidazolyl-3-yl),
o) eine, wahlweise substituierte, -(CH₂)₃- -Gruppe, die mit einer der zwei benachbarten Gruppen X einen Ring bildet, um die Seitenkette von Prolin, Hydroxyprolin oder Dehydroprolin zu bilden,
wobei die anderen Substituenten wie im Anspruch 1 definiert sind.

3. Verbindungen der Formel (I) nach Anspruch 2, wobei R₁, R₂, R₃ und R₄ die Seitenketten von Aminosäuren sind und aus einer der nachfolgenden Gruppen ausgewählt werden: Glycin, Alanin, Valin, Leucin, Isoleucin, Methionin, Phenylalanin, Tyrosin, Tryptophan, Prolin, Histidin, Norleucin, Norvalin, Alloisoleucin, Dehydroprolin, Hydroxyprolin, Cyclohexylglycin (Chg), α-Amino-n-buttersäure (Aba), Cyclohexylalanin (Cha), Aminophenylbuttersäure (Pba), Phenylalanin, das in Ortho-, Meta-, oder Para-Position des aromatischen Rings mit einer oder mehreren der nachfolgenden Gruppen mono- und disubstituiert ist:
C₁₋₁₀-Alkyl, C₁₋₁₀-Alcoxyl, Halogen, β-2-Thienylalanin, β-3-Thienylalanin, β-2-Furanylalanin, β-3-Furanylalanin, β-2-Pyridylalanin, β-3-Pyridylalanin, β-4-Pyridylalanin, β-(1-Naphthyl)alanin, β-(2-Naphthyl)alanin, O-alkylierte Derivate von Serin, Threonin, Tyrosin, S-alkyliertes Cystein, S-alkyliertes Homocystein, alkyliertes Lysin, alkyliertes Ornithin, 2,3-Diaminopropionsäure;
oder sie sind die Seitenketten von nicht-hydrophoben Aminosäuren, deren funktionelle Gruppen derivatisiert wurden, um sie hydrophob zu machen, ausgewählt aus der Gruppe, bestehend aus:
Serin, Threonin, Cystein, Asparaginsäure, Asparagin, Glutaminsäure, Glutamin, t-Carboxyglutaminsäure, Arginin, Ornithin, Lysin.

4. Verbindungen der Formel (I) nach Anspruch 3, wobei:
R₁ = -CH₂CH(CH₃)₂
R₂ = -CH₂C₆H₅ 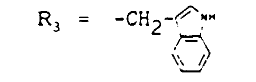
R₄ = -(CH₂)₂-SCH₃
X₁ = X₂ = X₃ = X₄ = X₅ = X₆ = -CONH-
Y = -CONH-
wobei die chiralen Kohlenstoffatome eine L-Konfiguration aufweisen.

5. Verbindungen der Formel (I) nach Anspruch 3, wobei:
Y = -NHCO-
und die anderen Substituenten wie im Anspruch 4 definiert sind.

6. Verbindungen der Formel (I) nach Anspruch 3, wobei:
R₄ = -CH₂-C₆H₁₁
und die anderen Substituenten wie im Anspruch 4 definiert sind.

7. Verbindungen der Formel (I) nach Anspruch 3, wobei:
Y = -NHCO-
und die anderen Substituenten wie im Anspruch 6 definiert sind.

8. Verbindungen der Formel (I) nach Anspruch 3, wobei:
R₂ = R₄ = -CH₂-C₆H₅ 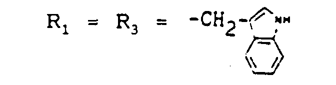
und die anderen Substituenten wie im Anspruch 4 definiert sind.

9. Verbindungen der Formel (I) nach Anspruch 3, wobei:
Y = -NHCO-
und die anderen Substituenten wie im Anspruch 8 definiert sind.

10. Verbindungen der Formel (I) nach Anspruch 3, wobei:
Y = -SS-
und die anderen Substituenten wie im Anspruch 4 definiert sind.

11. Verbindungen der Formel (I) nach Anspruch 3, wobei:
Y = -CH₂-CH₂-
und die anderen Substituenten wie im Anspruch 4 definiert sind.

12. Verbindungen der Formel (I) nach Anspruch 3, wobei:
Y = -CH=CH- (cis)
und die anderen Substituenten wie im Anspruch 4 definiert sind.

13. Verbindungen der Formel (I) nach Anspruch 3, wobei:
Y = -CH=CH- (trans)
und die anderen Substituenten wie im Anspruch 4 definiert sind.

14. Verbindungen der Formel (I) nach Anspruch 3, wobei:
Y = -CH₂NH-
und die anderen Substituenten wie im Anspruch 4 definiert sind.

15. Verbindungen der Formel (I) nach Anspruch 3, wobei:
Y = -NHCH₂-
und die anderen Substituenten wie im Anspruch 4 definiert sind.

16. Verbindungen der Formel (I) nach Anspruch 3, wobei:
m = n = 1
und die anderen Substituenten wie im Anspruch 4 definiert sind.

17. Verbindungen der Formel (I) nach Anspruch 3, wobei:
m = 2, n = 4
und die anderen Substituenten wie im Anspruch 4 definiert sind.

18. Verbindungen der Formel (I) nach Anspruch 3, wobei:
X₁ = X₂ = X₃ = X₄ = X₅ = X₆ = -NHCO-
und die anderen Substituenten wie im Anspruch 4 definiert sind.

19. Verbindungen der Formel (I) nach Anspruch 3, wobei:
Y = -NHCO-
und die anderen Substituenten wie im Anspruch 18 definiert sind.

20. Verbindungen der Formel (I) nach Anspruch 3, wobei:
R₄ = -CH₂-C₆H₁₁
und die anderen Substituenten wie im Anspruch 18 definiert sind.

21. Verbindungen der Formel (I) nach Anspruch 3, wobei:
Y = -NHCO-
und die anderen Substituenten wie im Anspruch 20 definiert sind.

22. Verbindungen der Formel (I) nach Anspruch 3, wobei:
R₂ = R₄ = -CH₂-C₆H₅ 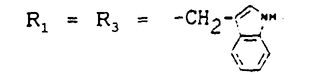
und die anderen Substituenten wie im Anspruch 18 definiert sind.

23. Verbindungen der Formel (I) nach Anspruch 3, wobei:
Y = -NHCO-
und die anderen Substituenten wie im Anspruch 22 definiert sind.

24. Verbindungen der Formel (I) nach Anspruch 3, wobei:
Y = -SS-
und die anderen Substituenten wie im Anspruch 18 definiert sind.

25. Verbindungen der Formel (I) nach Anspruch 3, wobei:
Y = -CH₂-CH₂-
und die anderen Substituenten wie im Anspruch 18 definiert sind.

26. Verbindungen der Formel (I) nach Anspruch 3, wobei:
Y = -CH=CH- (cis)
und die anderen Substituenten wie im Anspruch 18 definiert sind.

27. Verbindungen der Formel (I) nach Anspruch 3, wobei:
Y = -CH=CH- (trans)
und die anderen Substituenten wie im Anspruch 18 definiert sind.

28. Verbindungen der Formel (I) nach Anspruch 3, wobei:
m = n = 1
und die anderen Substituenten wie im Anspruch 18 definiert sind.

29. Verbindungen der Formel (I) nach Anspruch 3, wobei:
m = 1; n = 2
und die anderen Substituenten wie im Anspruch 18 definiert sind.

30. Verbindungen der Formel (I) nach Anspruch 3, wobei:
m = 1; n = 3
und die anderen Substituenten wie im Anspruch 18 definiert sind.

31. Verbindungen der Formel (I) nach Anspruch 3, wobei:
m = 1; n = 4
und die anderen Substituenten wie im Anspruch 18 definiert sind.

32. Verbindungen der Formel (I) nach Anspruch 3, wobei:
m = 2; n = 1
und die anderen Substituenten wie im Anspruch 18 definiert sind.

33. Verbindungen der Formel (I) nach Anspruch 3, wobei:
m = 2; n = 3
und die anderen Substituenten wie im Anspruch 18 definiert sind.

34. Verbindungen der Formel (I) nach Anspruch 3, wobei:
m = 2; n = 2
und die anderen Substituenten wie im Anspruch 18 definiert sind.

35. Verbindungen der Formel (I) nach Anspruch 3, wobei:
m = 2; n = 4
und die anderen Substituenten wie im Anspruch 18 definiert sind.

36. Verbindungen der Formel (I) nach Anspruch 3, wobei die Kohlenstoffatome in den Positionen 5 und 6 eine D-Konfiguration aufweisen und alle Substituenten wie im Anspruch 4 definiert sind.

37. Verbindungen der Formel (I) nach Anspruch 3, wobei alle chiralen Kohlenstoffatome eine D-Konfiguration aufweisen und alle Substituenten wie im Anspruch 4 definiert sind.

38. Pharmazeutische Zusammensetzungen, enthaltend Verbindungen der Formel (I) nach Anspruch 1, vermischt mit geeigneten Trägern.

39. Pharmazeutische Zusammensetzungen nach Anspruch 38 zur Verwendung als Tachyquinin-Antagonisten.

40. Zusammensetzungen nach Anspruch 38 zur Behandlung von Arthritis, Asthma, Entzündungen, Tumorwachstum, gastrointestinaler Hypermotilität, Chorea Huntington, Neuritis, Neuralgie, Migräne, Hypertonie, Urin-Inkontinenz, Urtikaria, Symtome eines karzinoiden Syndroms, Influenza und Erkältung.

## Patentansprüche (Patentansprüche für folgende(n) Vertragsstaat(en): ES)

1. Verfahren zur Herstellung von Verbindungen der allgemeinen Formel (I) 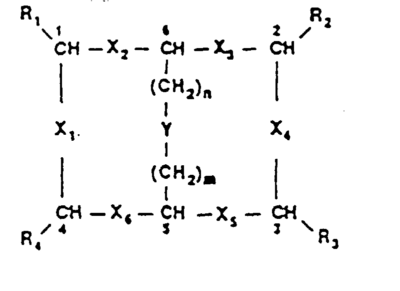 wobei
X₁, X₂, X₃, X₄, X₅ und X₆, die gleich oder verschieden sein können, je aus der Gruppe ausgewählt werden, bestehend aus -NR'-CO-, -CO-NR'-, wobei R' aus der Gruppe, bestehend aus H, C₁₋₃-Alkyl, ausgewählt wird;
Y ausgewählt wird aus der Gruppe, bestehend aus -CONR-, -NRCO-, -OCO-, -COO-, -CH₂-NR-, -NR-CH₂, -SS-, -CH₂-CH₂-, cis- oder trans- -CH=CH-, wobei R ausgewählt aus der Gruppe, bestehend aus H, C₁₋₃-Alkyl;
R₁, R₂, R₃ und R₄ je eine hydrophobe Gruppe sind;
n und m, die gleich oder verschieden sein können, je ein Ganzzahliges von 1 bis 4 sind,
wobei
eine Peptidkette in Festphase vom C-terminalen Ende zum N-terminalen Ende an einem unlöslichen Polymerträger synthetisiert wird, von der Peptidkette die Schutzgruppe entfernt wird und in der Festphase zyklisiert wird, worauf sie vom Polymerträger durch Hydrolyse in wasserfreier Fluorwasserstoffsäure oder Trifluoressigsäure, enthaltend Radikalfänger (scavengers), entfernt wird und schließlich das monozyklische Peptid in verdünnter Lösung organischer polarer Lösungsmittel zyklisiert wird.

2. Verfahren nach Anspruch 1, wobei R₁, R₂, R₃ und R₄ aus den nachfolgenden Gruppen ausgewählt werden:
a) linearen oder verzweigten Alkylgruppen des Typs CₙH₂ₙ₊₁, wobei n=O, 1 bis 4 ist;
b) linearen oder verzweigten Alkylgruppen des Typs CₙH₂ₙ-U-W, wobei n = 1 bis 4; U = O, CO, COO, CONH, S, Guanidin, NH und W=H, einer hydrophoben Gruppe mit 1 - 10 Kohlenstoffatomen;
c) CH₂C₆H₃XY, wobei X und Y, die gleich oder unterschiedlich sein können, je H, ein Halogen, OH, NH₂, CH₃ sind und in Ortho- oder Meta- oder Para-Stellung zum Benzolring stehen können;
d) CH₂C₆H₄X, wobei X = OR, SR, NHR, wobei R= eine hydrophobe Gruppe mit 1 - 10 Kohlenstoffatomen ist;
e) C₆H₃XY, wobei X und Y, die gleich oder verschieden sein können, je H, ein Halogen, OH, NH₂, CH₃ sind und in Ortho- oder Meta- oder Para-Position zum Benzolring stehen können;
f) CH₂C₆H₁₁
g) 1-Methyl-naphthyl, 2-Methyl-naphthyl,
h) CH₂-Imidazol,
i) CH₂-Indol,
l) CH₂-(Furanyl-3-yl),
m) CH₂-(Pyridyl-3-yl),
n) CH₂-(Imidazolyl-3-yl),
o) eine, wahlweise substituierte, -(CH₂)₃- -Gruppe, die mit einer der zwei benachbarten Gruppen X einen Ring bildet, um die Seitenkette von Prolin, Hydroxyprolin oder Dehydroprolin zu bilden,
wobei die anderen Substituenten wie im Anspruch 1 definiert sind.

3. Verfahren nach Anspruch 2, wobei R₁, R₂, R₃ und R₄ die Seitenketten von Aminosäuren sind und aus einer der nachfolgenden Gruppen ausgewählt werden: Glycin, Alanin, Valin, Leucin, Isoleucin, Methionin, Phenylalanin, Tyrosin, Tryptophan, Prolin, Histidin, Norleucin, Norvalin, Alloisoleucin, Dehydroprolin, Hydroxyprolin, Cyclohexylglycin (Chg), a-Amino-n-buttersäure (Aba), Cyclohexylalanin (Cha), Aminophenylbuttersäure (Pba), Phenylalanin, das in Ortho-, Meta-, oder Para-Position des aromatischen Rings mit einer oder mehreren der nachfolgenden Gruppen mono- und disubstituiert ist:
C₁₋₁₀-Alkyl, C₁₋₁₀-Alcoxyl, Halogen, β-2-Thienylalanin, β-3-Thienylalanin, β-2-Furanylalanin, β-3-Furanylalanin, β-2-Pyridylalanin, β-3-Pyridylalanin, β-4-Pyridylalanin, β-(1-Naphthyl)alanin, β-(2-Naphthyl)alanin, O-alkylierte Derivate von Serin, Threonin, Tyrosin, S-alkyliertes Cystein, S-alkyliertes Homocystein, alkyliertes Lysin, alkyliertes Ornithin, 2,3-Diaminopropionsäure;
oder sie sind die Seitenketten von nicht-hydrophoben Aminosäuren, deren funktionelle Gruppen derivatisiert wurden, um sie hydrophob zu machen, ausgewählt aus der Gruppe, bestehend aus:
Serin, Threonin, Cystein, Asparaginsäure, Asparagin, Glutaminsäure, Glutamin, t-Carboxyglutaminsäure, Arginin, Ornithin, Lysin.

4. Verfahren nach Anspruch 3, wobei in den Verbindungen der Formel (I):
R₁ = -CH₂CH(CH₃)₂
R₂ = -CH₂C₆H₅ 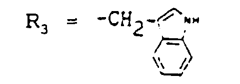
R₄ = -(CH₂)₂-SCH₃
X₁ = X₂ = X₃ = X, = X₅ = X₆ = -CONH-
Y = -CONH-
wobei die chiralen Kohlenstoffatome eine L-Konfiguration aufweisen.

5. Verfahren nach Anspruch 3, wobei in den Verbindungen der Formel (I):
Y = -NHCO-
und die anderen Substituenten wie im Anspruch 4 definiert sind.

6. Verfahren nach Anspruch 3, wobei in den Verbindungen der Formel (I):
R₄ = -CH₂-C₆H₁₁
und die anderen Substituenten wie im Anspruch 4 definiert sind.

7. Verfahren nach Anspruch 3, wobei in den Verbindungen der Formel (I):
Y = -NHCO-
und die anderen Substituenten wie im Anspruch 6 definiert sind.

8. Verfahren nach Anspruch 3, wobei in den Verbindungen der Formel (I):
R₂ = R₄ = -CH₂-C₆H₅ 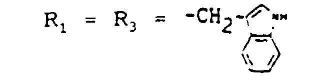
und die anderen Substituenten wie im Anspruch 4 definiert sind.

9. Verfahren nach Anspruch 3, wobei in den Verbindungen der Formel (I):
Y = -NHCO-
und die anderen Substituenten wie im Anspruch 8 definiert sind.

10. Verfahren nach Anspruch 3, wobei in den Verbindungen der Formel (I):
Y = -SS-
und die anderen Substituenten wie im Anspruch 4 definiert sind.

11. Verfahren nach Anspruch 3, wobei in den Verbindungen der Formel (I):
Y = -CH₂-CH₂-
und die anderen Substituenten wie im Anspruch 4 definiert sind.

12. Verfahren nach Anspruch 3, wobei in den Verbindungen der Formel (I):
Y = -CH=CH- (cis)
und die anderen Substituenten wie im Anspruch 4 definiert sind.

13. Verfahren nach Anspruch 3, wobei in den Verbindungen der Formel (I):
Y = -CH=CH- (trans)
und die anderen Substituenten wie im Anspruch 4 definiert sind.

14. Verfahren nach Anspruch 3, wobei in den Verbindungen der Formel (I):
Y = -CH₂NH-
und die anderen Substituenten wie im Anspruch 4 definiert sind.

15. Verfahren nach Anspruch 3, wobei in den Verbindungen der Formel (I):
Y = -NHCH₂-
und die anderen Substituenten wie im Anspruch 4 definiert sind.

16. Verfahren nach Anspruch 3, wobei in den Verbindungen der Formel (I):
m = n = 1
und die anderen Substituenten wie im Anspruch 4 definiert sind.

17. Verfahren nach Anspruch 3, wobei in den Verbindungen der Formel (I):
m = 2, n = 4
und die anderen Substituenten wie im Anspruch 4 definiert sind.

18. Verfahren nach Anspruch 3, wobei in den Verbindungen der Formel (I):
X₁ = X₂ = X₃ = X₄ = X₅ = X₆ = -NHCO-
und die anderen Substituenten wie im Anspruch 4 definiert sind.

19. Verfahren nach Anspruch 3, wobei in den Verbindungen der Formel (I):
Y = -NHCO-
und die anderen Substituenten wie im Anspruch 18 definiert sind.

20. Verfahren nach Anspruch 3, wobei in den Verbindungen der Formel (I):
R₄ = -CH₂-C₆H₁₁
und die anderen Substituenten wie im Anspruch 18 definiert sind.

21. Verfahren nach Anspruch 3, wobei in den Verbindungen der Formel (I):
Y = -NHCO-
und die anderen Substituenten wie im Anspruch 20 definiert sind.

22. Verfahren nach Anspruch 3, wobei in den Verbindungen der Formel (I):
R₂ = R₄ = -CH₂-C₆H₅ 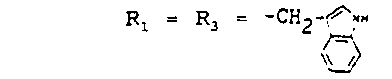
und die anderen Substituenten wie im Anspruch 18 definiert sind.

23. Verfahren nach Anspruch 3, wobei in den Verbindungen der Formel (I):
Y = -NHCO-
und die anderen Substituenten wie im Anspruch 22 definiert sind.

24. Verfahren nach Anspruch 3, wobei in den Verbindungen der Formel (I):
Y = -SS-
und die anderen Substituenten wie im Anspruch 18 definiert sind.

25. Verfahren nach Anspruch 3, wobei in den Verbindungen der Formel (I):
Y = -CH₂-CH₂-
und die anderen Substituenten wie im Anspruch 18 definiert sind.

26. Verfahren nach Anspruch 3, wobei in den Verbindungen der Formel (I):
Y = -CH=CH- (cis)
und die anderen Substituenten wie im Anspruch 18 definiert sind.

27. Verfahren nach Anspruch 3, wobei in den Verbindungen der Formel (I):
Y = -CH=CH- (trans)
und die anderen Substituenten wie im Anspruch 18 definiert sind.

28. Verfahren nach Anspruch 3, wobei in den Verbindungen der Formel (I ):
m = n = 1
und die anderen Substituenten wie im Anspruch 18 definiert sind.

29. Verfahren nach Anspruch 3, wobei in den Verbindungen der Formel (I):
m = 1; n = 2
und die anderen Substituenten wie im Anspruch 18 definiert sind.

30. Verfahren nach Anspruch 3, wobei in den Verbindungen der Formel (I):
m = 1; n = 3
und die anderen Substituenten wie im Anspruch 18 definiert sind.

31. Verfahren nach Anspruch 3, wobei in den Verbindungen der Formel (I):
m = 1; n = 4
und die anderen Substituenten wie im Anspruch 18 definiert sind.

32. Verfahren nach Anspruch 3, wobei in den Verbindungen der Formel (I):
m = 2; n = 1
und die anderen Substituenten wie im Anspruch 18 definiert sind.

33. Verfahren nach Anspruch 3, wobei in den Verbindungen der Formel (I):
m = 2; n = 3
und die anderen Substituenten wie im Anspruch 18 definiert sind.

34. Verfahren nach Anspruch 3, wobei in den Verbindungen der Formel (I):
m = 2; n = 2
und die anderen Substituenten wie im Anspruch 18 definiert sind.

35. Verfahren nach Anspruch 3, wobei in den Verbindungen der Formel (I):
m = 2; n = 4
und die anderen Substituenten wie im Anspruch 18 definiert sind.

36. Verfahren nach Anspruch 3, wobei in den Verbindungen der Formel (I)
die Kohlenstoffatome in den Positionen 5 und 6 eine D-Konfiguration aufweisen und alle Substituenten wie im Anspruch 4 definiert sind.

37. Verfahren nach Anspruch 3, wobei in den Verbindungen der Formel (I) alle chiralen Kohlenstoffatome eine D-Konfiguration aufweisen und alle Substituenten wie im Anspruch 4 definiert sind.

38. Pharmazeutische Zusammensetzungen, enthaltend Verbindungen der Formel (I) nach Anspruch 1, vermischt mit geeigneten Trägern.

39. Pharmazeutische Zusammensetzungen nach Anspruch 38 zur Verwendung als Tachyquinin-Antagonisten.

40. Zusammensetzungen nach Anspruch 38 zur Behandlung von Arthritis, Asthma, Entzündungen, Tumorwachstum, gastrointestinaler Hypermotilität, Chorea Huntington, Neuritis, Neuralgie, Migräne, Hypertonie, Urin-Inkontinenz, Urtikaria, Symtome eines karzinoiden Syndroms, Influenza und Erkältung.

## Revendications (Revendications pour l'(les) Etat(s) contractant(s) suivant(s): AT, BE, CH, DE, DK, FR, GB, GR, IE, IT, LI, LU, MC, NL, PT, SE)

1. Produits de formule générale (I) 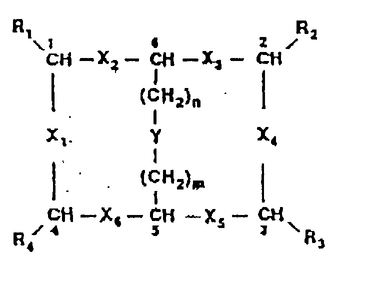 dans laquelle:
X₁, X₂, X₃, X₄, X₅ et X₆, identiques ou différents, sont chacun choisis dans l'ensemble constitué par les groupes -NR'-CO-, -CO-NR'-, où R' est choisi dans l'ensemble constitué par H, un groupe alkyle en C₁-C₃,
Y est choisi dans l'ensemble constitué par les groupes -CONR-, -NRCO-, -OCO-, -COO-, -CH₂-NR-, -NRCH₂-, -SS-, -CH₂-CH₂-, -CH=CH- cis ou trans, où R est choisi dans l'ensemble constitué par H, un groupe alkyle en C₁-C₃, R₁, R₂, R₃ et R₄ représentent chacun un groupe hydrophobe
n et m, identiques ou différents, représentent chacun un nombre entier de 1 à 4.

2. Composés de formule (I) selon la revendication 1, dans laquelle R₁, R₂, R₃ et R₄ sont choisis parmi les groupes suivants:
a) les groupes alkyle linéaires ou ramifiés du type CₙH₂ₙ₊₁, où n = 0, 1 à 4
b) les groupes alkyle linéaires ou ramifiés du type CₙH₂ₙ-U-W, X où n = 1 à 4; U = O, CO, COO, CONH, S guanidine, NH, et W = H, un groupe hydrophobe contenant 1 à 10 atomes de carbone
c) CH₂C₆H₃XY, où X et Y, identiques ou différents, représentent chacun H, un atome d'halogène, OH, NH₂, CH₃, en position ortho ou méta ou para du noyau benzénique
d) CH₂C₆H₄X, où X = OR, SR, NHR, où R = un groupe hydrophobe contenant 1 à 10 atomes de carbone
e) C₆H₃XY, où X et Y, identiques ou différents, représentent chacun H, un atome d'halogène, OH, NH₂, CH₃, en position ortho ou méta ou para du noyau benzénique
f) CH₂C₆H₁₁
g) 1-méthylnaphtyle, 2-méthylnaphtyle
h) CH₂-imidazole
i) CH₂-indole
1) CH₂-(furanyl-3-yle)
m) CH₂-(pyridyl-3-yle)
n) CH₂-(imidazolyl-3-yle)
o) un groupe -(CH₂)₃- éventuellement substitué, qui forme un cycle avec l'un des deux groupes X adjacents pour donner la chaîne latérale de la proline, de l'hydroxyproline, de la déhydroproline
les autres substituants étant tels que définis dans la revendication 1.

3. Composés de formule (I) selon la revendication 2, dans laquelle R₁, R₂, R₃ et R₄ représentent les chaînes latérales d'acides aminés choisis dans le groupe constitué par: la glycine, l'analine, la valine, la leucine, l'isoleucine, la méthionine, la phénylalanine, la tyrosine, le tryptophane, la proline, l'histidine, la norleucine, la norvaline, l'alloisoleucine, la déhydroproline, l'hydroxyproline, la cyclohexylglycine (Chg), l'acide α-amino-n-butyrique (Aba), la cyclohexylalanine (Cha), l'acide aminophénylbutyrique (Pba), la phénylalanine mono- et di-substituée en position ortho, méta ou para du noyau aromatique par un ou plusieurs des groupes suivants: alkyle en C₁-C₁₀, alcoxy en C₁-C₁₀, un atome d'halogène, la β-2-thiénylalanine, la β-3-thiénylalanine, la β-2-furanylalanine, la β-3-furylalanine, la β-2-pyridylalanine, la β-3-pyridylalanine, la β-4-pyridylalanine, la β-(1-naphtyl)alanine, la β-(2-naphtyl)alanine, les dérivés O-alkylés de la sérine, de la thréonine, de la tyrosine, de la cystéine S-alkylée, de l'homocystéine S-alkylée, de la lysine alkylée, de l'ornithine alkylée, de l'acide 2,3-diaminopropionique; ou ils représentent les chaînes latérales d'acides aminés non hydrophobes dont les groupes fonctionnels sont dérivés pour les rendre hydrophobes, choisis dans le groupe constitué par: la sérine, la thréonine, la cystéine, l'acide aspartique, l'asparagine, l'acide glutamique, la glutamine, l'acide t-carboxyglutamique, l'arginine, l'ornithine, la lysine.

4. Composés de formule (I) selon la revendication 3, dans laquelle:
R₁ = -CH₂CH(CH₃)₂
R₂ = -CH₂C₆H₅ 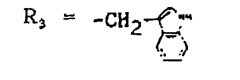
R₄ = -(CH₂)₂-SCH₃
X₁ = X₂ = X₃ = X₄ = X₅ = X₆ = -CONH
Y = -CONH-
où les atomes de carbone chiraux presentent une configuration L.

5. Composés de formule (I) selon la revendication 3, dans laquelle:
Y = -NHCO-
les autres substituants étant tels que définis dans la revendication 4.

6. Composés de formule (I) selon la revendication 3, dans laquelle:
R₄ = -CH₂-C₆H₁₁
les autres substituants étant tels que définis dans la revendication 4.

7. Composés de formule (I) selon la revendication 3, dans laquelle:
Y = -NHCO-
les autres substituants étant tels que définis dans la revendication 6.

8. Composés de formule (I) selon la revendication 3, dans laquelle:
R₂ = R₄ = -CH₂-C₆H₅ 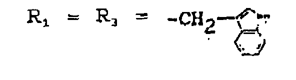
les autres substituants étant tels que définis dans la revendication 4.

9. Composés de formule (I) selon la revendication 3, dans laquelle:
Y= -NHCO-
les autres substituants étant tels que définis dans la revendication 8.

10. Composés de formule (I) selon la revendication 3, dans laquelle:
Y= -SS-
les autres substituants étant tels que définis dans la revendication 4.

11. Composés de formule (I) selon la revendication 3, dans laquelle:
Y = -CH₂-CH₂-
les autres substituants étant tels que définis dans la revendication 4.

12. Composés de formule (I) selon la revendication 3, dans laquelle:
Y = -CH-CH- (cis)
les autres substituants étant tels que définis dans la revendication 4.

13. Composés de formule (I) selon la revendication 3, dans laquelle:
Y = -CH=CH- (trans)
les autres substituants étant tels que définis dans la revendication 4.

14. Composés de formule (I) selon la revendication 3, dans laquelle:
Y = -CH₂ NH-
les autres substituants étant tels que définis dans la revendication 4.

15. Composés de formule (I) selon la revendication 3, dans laquelle:
Y = -NH CH₂-
les autres substituants étant tels que définis dans la revendication 4.

16. Composés de formule (I) selon la revendication 3, dans laquelle:
m = n = 1
les autres substituants étant tels que définis dans la revendication 4.

17. Composés de formule (I) selon la revendication 3, dans laquelle:
m = 2, n = 4
les autres substituants étant tels que définis dans la revendication 4.

18. Composés de formule (I) selon la revendication 3, dans laquelle:
X₁ = X₂ = X₃ = X₄ = X₅ = X₆ = -NHCO-
les autres substituants étant tels que définis dans la revendication 4.

19. Composés de formule (I) selon la revendication 3, dans laquelle:
Y = -NHCO-
les autres substituants étant tels que définis dans la revendication 18.

20. Composés de formule (I) selon la revendication 3, dans laquelle:
R₄ = -CH₂C₆H₁₁
les autres substituants étant tels que définis dans la revendication lB.

21. Composés de formule (I) selon la revendication 3, dans laquelle:
Y = -NHCO-
les autres substituants étant tels que définis dans la revendication 20.

22. Composés de formule (I) selon la revendication 3, dans laquelle:
R₂ = R₄ = -CH₂-C₆H₅ 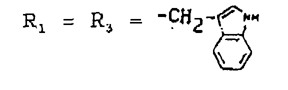
les autres substituants étant tels que définis dans la revendication 18.

23. Composés de formule (I) selon la revendication 3, dans laquelle:
Y = -NHCO-
les autres substituants étant tels que définis dans la revendication 22.

24. Composés de formule (I) selon la revendication 3, dans laquelle:
Y = -SS-
les autres substituants étant tels que définis dans la revendication 18.

25. Composés de formule (I) selon la revendication 3, dans laquelle:
Y = -CH₂-CH₂-
les autres substituants étant tels que définis dans la revendication 18.

26. Composés de formule (I) selon la revendication 3, dans laquelle:
Y = -CH=CH- (cis)
les autres substituants étant tels que définis dans la revendication 18.

27. Composés de formule (I) selon la revendication 3, dans laquelle:
Y = -CH=CH- (trans)
les autres substituants étant tels que définis dans la revendication 18.

28. Composés de formule (I) selon la revendication 3, dans laquelle:
m = n = 1
les autres substituants étant tels que définis dans la revendication 18.

29. Composés de formule (I) selon la revendication 3, dans laquelle:
m = 1, n = 2
les autres substituants étant tels que définis dans la revendication 18.

30. Composés de formule (I) selon la revendication 3, dans laquelle:
m = 1, n = 3
les autres substituants étant tels que définis dans la revendication 18.

31. Composés de formule (I) selon la revendication 3, dans laquelle:
m = 1, n = 4
les autres substituants étant tels que définis dans la revendication 18.

32. Composés de formule (I) selon la revendication 3, dans laquelle:
m = 2, n = 1
les autres substituants étant tels que définis dans la revendication 18.

33. Composés de formule (I) selon la revendication 3, dans laquelle:
m = 2, n = 3
les autres substituants étant tels que définis dans la revendication 18.

34. Composés de formule (I) selon la revendication 3, dans laquelle:
m = 2, n = 2
les autres substituants étant tels que définis dans la revendication 18.

35. Composés de formule (I) selon la revendication 3, dans laquelle:
m = 2, n = 4
les autres substituants étant tels que définis dans la revendication 18.

36. Composés de formule (I) selon la revendication 3, dans laquelle les atomes de carbone en positions 5 et 6 présentent une configuration D, tous les substituants étant tels que définis dans la revendication 4.

37. Composés de formule (I) selon la revendication 3, dans laquelle les atomes de carbone chiraux présentent une configuration D, tous les substituants étant tels que définis dans la revendication 4.

38. Compositions pharmaceutiques contenant les composés de formule (I) selon la revendication 1 mélangés avec des véhicules convenables.

39. Compositions pharmaceutiques selon la revendication 38 à utiliser comme antagonistes de la tachyquinine.

40. Compositions selon la revendication 38 pour le traitement de l'arthrite, de l'asthme, des inflammations, d'une croissance tumorale, de l'hypermotilité gastro-intestinale, de la maladie d'Huntington, de la neuvrite, de la neuralgie, de la migraine, de l'hypertension, de l'incontinence urinaire, de l'urticaire, des symptômes du syndrome carcinoïde, de la grippe et du rhume.

## Revendications (Revendications pour l'(les) Etat(s) contractant(s) suivant(s): ES)

1. Procédé de préparation de produits de formule générale (I) 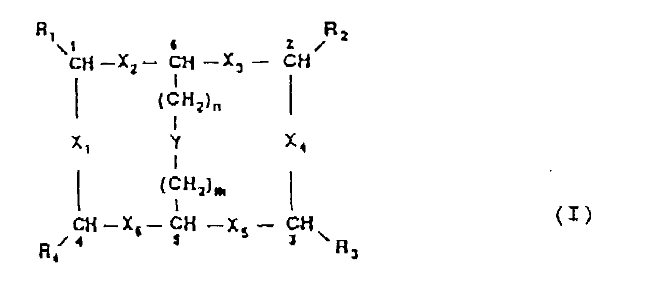 dans laquelle:
X₁, X₂, X₃, X₄, X₅ et X₆, identiques ou différents, sont chacun choisis dans l'ensemble constitué par les groupes -NR'-CO-, -CO-NR'-, où R' est choisi dans l'ensemble constitué par H, un groupe alkyle en C₁-C₃, Y est choisi dans l'ensemble constitué par les groupes -CONR-; -NRCO-, -OCO-, -COO-, -CH₂-NR-, -NR-CH₂-, -SS-, -CH₂-CH₂-, -CH=CH- cis ou trans, où R est choisi dans l'ensemble constitué par H, un groupe alkyle en C₁-C₃, R₁, R₂, R₃ et R₄ représentent chacun un groupe hydrophobe
n et m, identiques ou différents, représentent chacun un nombre entier de 1 à 4.
dans lequel:
une chaîne peptidique est synthétisée en phase solide à j partir d'une extrémité C-terminale pour donner une extrémité N-terminale sur un support de polymère insoluble, la chaîne peptidique est déprotégée et cyclisée en phase solide, puis détachée du support de polymère par hydrolyse dans de l'acide fluorhydrique anhydre ou l'acide trifluoroacétique, contenant des épurateurs convenables, et enfin le peptide monocyclique est cyclisé dans une solution diluée de solvants organiques polaires.

2. Procédé selon la revendication 1, dans lequel R₁, R₂, R₃ et R₄ sont choisis parmi les groupes suivants:
a) les groupes alkyle linéaires ou ramifiés du type CₙH₂ₙ₊₁, où n = 0, 1 à 4
b) les groupes alkyle linéaires ou ramifiés du type CₙH₂ₙ-U-W, où n = 1 à 4; U = O, CO, COO, CONH, S guanidine, NH, et W = H, un groupe hydrophobe contenant 1 à 10 atomes de carbone
c) CH₂C₆H₃XY, où X et Y, identiques ou différente, représentent chacun H, un atome d'halogène, OH, NH₂, CH₃, en position ortho ou méta ou para du noyau benzénique
d) CH₂C₆H₄X, où X = OR, SR, NHR, où R = un groupe hydrophobe contenant 1 à 10 atomes de carbone
e) C₆H₃XY, où X et Y, identiques ou différente, représentent chacun H, un atome d'halogène, OH, NH₂, CH₃, en position ortho ou méta ou para du noyau benzénique
f) CH₂C₆H₁₁
g) 1-methylnaphtyle, 2-méthylnaphtyle
h) CH₂-imidazole
i) CH₂-indole
l) CH₂-(furanyl-3-yle)
m) CH₂-(pyridyl-3-yle)
n) CH₂-(imidazolyl-3-yle)
o) un groupe - (CH₂)₃- éventuellement substitué, qui forme un cycle avec l'un des deux groupes x adjacents pour donner la chaîne latérale de la proline, de l'hydroxyproline, de la déhydroproline
les autres substituants étant tels que définis dans la revendication 1.

3. Procédé selon la revendication 2, dans lequel R₁, R₂, R₃ et R₄ représentent les chaînes latérales d'acides aminés choisis dans le groupe constitué par: la glycine, l'analine, la valine, la leucine, l'isoleucine, la méthionine, la phénylalanine, la tyrosine, le tryptophane, la proline, l'histidine, la norleucine, la norvaline, l'alloieoleucine, la déhydroproline, l'hydroxyproline, la cyclohexylglycine (Chg), l'acide α-amino-n-butyrique (Aba), la cyclohexylalanine (Cha), l'acide aminophénylbutyrique (Pba), la phénylalanine mono- et di-substituée en position ortho, méta ou para du noyau aromatique par un ou plusieurs des groupes suivants: alkyle en C₁-C₁₀, alcoxy en C₁-C₁₀, un atome d'halogène, la β-2-thiénylalanine, la β-3-thiénylalanine, la β-2-furanylalanine, la β-3-furylalanine, la β-2-pyridylalanine, la β-3-pyridylalanine, la β-4-pyridylalanine, la β-(1-naphtyl)alanine, la β-(2-naphtyl)alanine, les dérivée O-alkylés de la sérine, de la thréonine, de la tyrosine, de la cystéine S-alkylée, de l'homocystéine S-alkylée, de la lysine alkylée, de l'ornithine alkylée, de l'acide 2,3-diaminopropionique; ou ils représentent les chaînes latérales d'acides aminés non hydrophobes dont les groupes fonctionnels sont dérivés pour les rendre hydrophobes, choisis dans le groupe constitué par: la sérine, la thréonine, la cystéine, l'acide aspartique, l'asparagine, l'acide glutamique, la glutamine, l'acide t-carboxyglutamique, l'arginine, l'ornithine, la lysine.

4. Procédé selon la revendication 3, dans lequel dans les composés de formule (I) :
R₁ = -CH₂CH(CH₃)₂
R₂ = -CH₃C₆H₅ 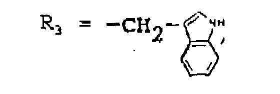
R₄ = -(CH₂)₂-SCH₃
X₁ = X₂ = X₃ = X₄ = X₅ = X₆ = -CONH-
Y = -CONH-
où les atomes de carbone chiraux présentent une configuration L.

5. Procédé selon la revendication 3, dans lequel dans les composés de formule (I) :
Y = -NHCO-
les autres substituants étant tels que définis dans la revendication 4.

6. Procédé selon la revendication 3, dans lequel dans les composés de formule (I) :
R₄ = -CH₂-C₆H₁₁
les autres substituants étant tels que définis dans la revendication 4.

7. Procédé selon la revendication 3, dans lequel dans les composés de formule (I) :
Y = -NHCO-
les autres substituants étant tels que définis dans la revendication 6.

8. Procédé selon la revendication 3, dans lequel dans les composés de formule (I) :
R₂ = R₄ = -CH₂-C₆H₅ 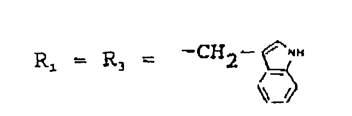
les autres substituants étant tels que définis dans la revendication 4.

9. Procédé selon la revendication 3, dans lequel dans les composés de formule (I):
Y = -NHCO-
les autres substituants étant tels que définis dans la revendication 8.

10. Procédé selon la revendication 3, dans lequel dans les composés de formule (I) :
Y = -SS-
les autres substituants étant tels que définis dans la revendication 4.

11. Procédé selon la revendication 3, dans lequel dans les composés de formule (I) :
Y = -CH₂-CH₂-
les autres substituants étant tels que définis dans la revendication 4.

12. Procédé selon la revendication 3, dans lequel dans les composés de formule (I) :
Y = -CH=CH- (cis)
les autres substituants étant tels que définis dans la revendication 4.

13. Procédé selon la revendication 3, dans lequel dans les composés de formule (I):
Y = -CH=CH- (trans)
les autres substituants étant tels que définis dans la revendication 4.

14. Procédé selon la revendication 3, dans lequel dans les composés de formule (I):
Y = -CH₂-NH-
les autres substituants étant tels que définis dans la revendication 4.

15. Procédé selon la revendication 3, dans lequel dans les composés de formule (I):
Y = -NH-CH₂-
les autres substituants étant tels que définis dans la revendication 4.

16. Procédé selon la revendication 3, dans lequel dans les composés de formule (I):
m = n = 1
les autres substituants étant tels que définis dans la revendication 4.

17. Procédé selon la revendication 3, dans lequel dans les composés de formule (I):
m = 2, n = 4
les autres substituants étant tels que définis dans la revendication 4.

18. Procédé selon la revendication 3, dans lequel dans les composés de formule (I) :
X₁ = X₂ = X₃ = X₄ = X₅ = X₆ = -NHCO-
les autres substituants étant tels que définis dans la revendication 4.

19. Procédé selon la revendication 3, dans lequel dans les composés de formule (I) :
Y = -NHCO-
les autres substituants étant tels que définis dans la revendication 18.

20. Procédé selon la revendication 3, dans lequel dans les composés de formule (I) :
R₄ = -CH₂-C₆H₁₁
les autres substituants étant tels que définis dans la revendication 18.

21. Procédé selon la revendication 3, dans lequel dans les composés de formule (I) :
Y = -NHCO-
les autres substituants étant tels que définis dans la revendication 20.

22. Procédé selon la revendication 3, dans lequel dans les composés de formule (I):
R₂ = R₄ = -CH₂-C₆H₅ 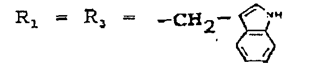
les autres substituants étant tels que définis dans la revendication 18.

23. Procédé selon la revendication 3, dans lequel dans les composés de formule (I):
Y = -NHCO-
les autres substituants étant tels que définis dans la revendication 22.

24. Procédé selon la revendication 3, dans lequel dans les composés de formule (I) :
Y = -SS-
les autres substituants étant tels que définis dans la revendication 18.

25. Procédé selon la revendication 3, dans lequel dans les composés de formule (I) :
Y = -CH₂-CH₂-
les autres substituants étant tels que définis dans la revendication 18.

26. Procédé selon la revendication 3, dans lequel dans les composés de formule (I) :
Y = -CH=CH- (cis)
les autres substituants étant tels que définis dans la revendication 18.

27. Procédé selon la revendication 3, dans lequel dans les composes de formule (I):
Y = -CH-CH- (trans)
les autres substituants étant tels que définis dans la revendication 18.

28. Procédé selon la revendication 3, dans lequel dans les composés de formule (I) :
m = n = 1
les autres substituants étant tels que définis dans la revendication 18.

29. Procédé selon la revendication 3, dans lequel dans les composés de formule (I) :
m = 1, n = 2
les autres substituants étant tels que définis dans la revendication 18.

30. Procédé selon la revendication 3, dans lequel dans les composés de formule (I) :
m = 1, n = 3
les autres substituants étant tels que définis dans la revendication 18.

31. Procédé selon la revendication 3, dans lequel dans les composés de formule (I):
m = 1, n = 4
les autres substituants étant tels que définis dans la revendication 18.

32. Procédé selon la revendication 3, dans lequel dans les composés de formule (I):
m = 2, n = 1
les autres substituants étant tels que définis dans la revendication 18.

33. Procédé selon la revendication 3, dans lequel dans les composés de formule (I) :
m = 2, n = 3
les autres substituants étant tels que définis dans la revendication 18.

34. Procédé selon la revendication 3, dans lequel dans les composés de formule (I):
m = 2, n = 2
les autres substituants étant tels que définis dans la revendication 18.

35. Procédé selon la revendication 3, dans lequel dans les composés de formule (I):
m = 2, n = 4
les autres substituants étant tels que définie dans la revendication 18.

36. Procédé selon la revendication 3, dans lequel dans les composés de formule (I) les atomes de carbone en positions 5 et 6 présentent une configuration D, tous les substituants étant tels que définis dans la revendication 4.

37. Procédé selon la revendication 3, dans lequel dans les composés de formule (I), les atomes de carbone chiraux présentent une configuration D, tous les substituants étant tels que définis dans la revendication 4.

38. Compositions pharmaceutiques contenant les composés de formule (I) selon la revendication 1 mélangés avec des véhicules convenables tels que décrits dans le présent document.

39. Compositions pharmaceutiques selon la revendication 38 à utiliser comme antagonistes de la tachyquinine.

40. Compositions selon la revendication 38 pour le traitement de l'arthrite, de l'asthme, des inflammations, d'une croissance tumorale, de l'hypermotilité gastro-intestinale, de la maladie d'Huntington, de la neuvrite, de la neuralgie, de la migraine, de l'hypertension, de l'incontinence urinaire, de l'urticaire, des symptômes du syndrome carcinoïde, de la grippe et du rhume.
